# EUROPÄISCHE PATENTSCHRIFT

(11) **EP 1 550 512 B1**
(45) Veröffentlichungstag und Bekanntmachung des Hinweises auf die Patenterteilung: **16.09.2009**
(21) Anmeldenummer: 04405718.0
(22) Anmeldetag: 22.11.2004
(51) Int. Cl.: B05C 17/005

(54) **Auftragvorrichtung für ein Austraggerät**
Fluid applying device for a fluid dispensing apparatus
Dispositif d'application de fluide pour un appareil de distribution de fluide

(30) Priorität: 12.12.2003 CH 21222003
(43) Veröffentlichungstag der Anmeldung: 06.07.2005
(73) Patentinhaber: Medmix Systems AG, 6343 Rotkreuz (CH)
(72) Erfinder: Keller, Wilhelm A., 6402 Merlischachen (CH)
(74) Vertreter: AMMANN PATENTANWÄLTE AG BERN

(56) Entgegenhaltungen:
- US-A- 1 038 180
- US-A- 3 767 085

## Beschreibung

Die vorliegende Erfindung bezieht sich auf eine Auftragvorrichtung für ein Austraggerät gemäss dem Oberbegriff von Anspruch 1, insbesondere für eine Kartusche und ist für die Medizinaltechnik zum Verkleben von Gewebe wie beispielsweise Lunge oder Leber gedacht.

Das Verkleben von Geweben wie Lunge, Leber oder Milz erlangt in der Medizin einen stetig wachsenden Stellenwert, doch sind bis jetzt keine dafür speziell ausgebildeten Vorrichtungen bekannt, während herkömmliche Vorsätze an Mischern keine befriedigende Lösung darstellen.

Das gilt auch im grösseren Massstab für das Verkleben, Beschichten oder Versiegeln von Stoffen, Zeltplanen, geschäumten Teilen oder dergleichen Materialien mit unebener Oberfläche.

Aus der US-A-1 038 180 ist eine Auftragvorrichtung gemäss Oberbegriff von Patentanspruch 1 bekannt, die für das Auftragen von Mörtel und dergleichen gedacht ist und ein gerades, der Wand zugewandtes Teil aufweist, das eine fast die gesamte Unterseite einnehmende Mörtelöffnung aufweist. Eine solche Vorrichtung ist für das Verkleben von Gewebeteilen nicht geeignet, da die grosse Öffnung ein genaues Dosieren und Verkleben sowie Glätten nicht erlaubt.

US-A-2 804 767 offenbart eine Vorrichtung, die für das Auftragen von Gips, Zement oder Mörtel gedacht ist und ein abgewinkeltes Auftragteil aufweist, wobei die Oberseite des Auftragteils dessen Unterseite wesentlich überragt, womit eine relativ grosse Menge des Materials herausströmen kann. Dies ist jedoch für die gedachte Verwendung nicht geeignet.

Aus der EP-A-1 157 748 ist ein Leimgerät bekannt mit einem Auslassteil, dessen Unterseite einen Knick aufweist.

Diese vorbekannten Geräte sind zum Verkleben oder definierten Beschichten von unebenen, empfindlichen und nachgebenden Oberflächen in der Medizinaltechnik, wie Lunge oder dergleichen, nicht geeignet, da das Hauptproblem dort darin besteht, dass eine solche Unterlage sehr uneben und weich ist, wodurch ein effizientes und gleichmässiges Auftragen von Klebstoff sehr problematisch ist.

Davon ausgehend ist es Aufgabe der vorliegenden Erfindung eine verbesserte Auftragvorrichtung für ein Austraggerät anzugeben, mit der ein effizientes und schonendes Auftragen von Kleb- oder Dichtstoff in der Medizinaltechnik, auf Gewebe wie Lunge, Leber oder dergleichen möglich ist. Eine Auftragvorrichtung, die diese Aufgabe löst, ist in Patentanspruch 1 definiert.

Die Erfindung wird im Folgenden anhand von Zeichnungen von Ausführungsbeispielen näher erläutert.
- Fig. 1: zeigt eine erfindungsgemässe Auftragvorrichtung von oben,
- Fig. 2: zeigt einen Schnitt gemäss Linie II-II in Fig. 1,
- Fig. 2A: zeigt in einer Ausschnittsvergrösserung eine Ausführungsvariante des Auslassendes,
- Fig. 3: zeigt einen Schnitt gemäss Linie II-II in Fig. 2,
- Figuren 4: zeigt eine Ausführungsform des Gehäuses der Auftragvorrichtung, die
- Figuren 5A bis 5C: zeigen drei Ausführungsvarianten bezüglich der Form des Auslassendes, ünd die
- Figuren 6A - 6G: zeigen Ausführungsvarianten bezüglich des Austrittsquerschnitts.

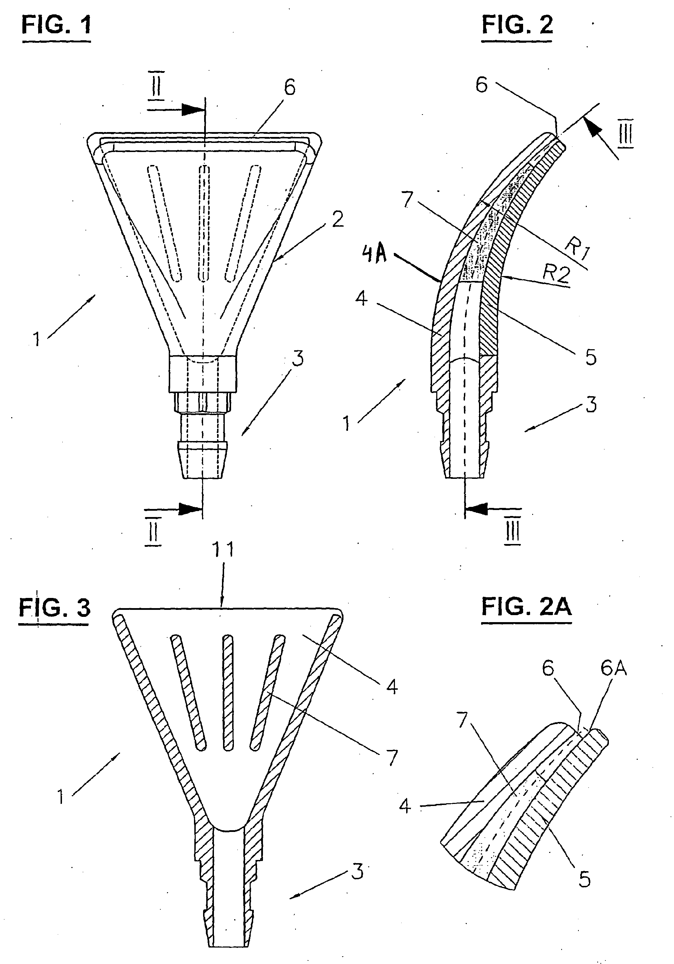

Die Auftragvorrichtung 1 besteht aus einem zweiteiligen Gehäuse 2 mit Anschlussteil 3, wobei das Gehäuse 2 aus dem Unterteil 4 und dem deckelförmigen Oberteil 5 besteht. Aus Fig. 1 geht hervor, dass sich das Gehäuse 2 konisch nach Aussen, das heisst zum Austragschlitz 6 hin, verbreitert.

Im Ausführungsbeispiel gemäss den Figuren 1 bis 3 kann die Schlitzbreite 12 oder 16 mm und die Schlitzdicke 0,1 - 0,3 mm betragen. Die untere und obere Seite des Gehäuses wird in Bezug auf die zu klebenden Teile definiert, wobei Gehäuseteil 4 auf das Gewebe, beispielsweise Lungengewebe, zu liegen kommt und daher das untere Teil und Teil 5 demgemäss das obere Teil ist.

Wie aus Fig. 2A hervorgeht, kann das obere Teil 5 auch etwas länger als das untere Teil 4 sein, wobei der Unterschied 6A in vorliegendem Ausführungsbeispiel 0,5 mm beträgt. Damit kann in gewissen Fällen erreicht werden, dass der aus dem Austragschlitz 6 austretende Klebstoff sich zum Gewebe hin wendet.

Aus Figur 2 geht ferner hervor, dass mindestens die Gehäuseunterseite 4A gekrümmt ist, wobei diese Krümmung wie gezeichnet stetig, kreisbogenförmig und mit einem Radius definiert werden kann, eine andere Krümmung wie elliptisch oder parabelförmig aufweisen kann. Wichtig ist dabei, dass die Längsachse des Anschlussteils 3 mit der zu verklebenden Oberfläche, bzw. der Gehäuseunterseite 4A, einen Winkel bildet, der je nach Anwendungszweck verschieden sein kann. Die Glättfunktion hängt auch von der Oberflächenbeschaffenheit und dem verwendeten Material der Gehäuseunterseite ab.

In Fig. 3 ist der untere Teil 4 des Gehäuses von innen sichtbar, wobei hier drei Leitstege 7 eingezeichnet sind. Diese Leitstege haben die Aufgabe, den vom Anschlussteil her kommenden Klebstoff zu verteilen, um einen gleichmässigen Ausfluss zu erzielen.

Das Anschlussteil 3 ist ausgebildet, in den Auslass eines Mischers gesteckt zu werden, doch sind selbstverständlich Anschlussteile mit anderen Dimensionen und Ausgestaltungen möglich, um eine Verbindung zu einem bestimmten Mischer oder Austraggerät herstellen zu können.

Das Gehäuse 2 ist wie eingangs erwähnt, zweiteilig und wird auch zweiteilig hergestellt, wobei auf das untere Teil 4 gemäss Fig. 3 das obere Teil 5, das als Deckel ausgebildet ist, aufgesteckt und mit dem Unterteil verbunden wird. Die Verbindung kann auf vielfältige Art, beispielsweise auch durch Kleben oder Ultraschallschweissen hergestellt werden.

Beim Auftragen eines Klebstoffes auf ein Gewebe mit unebener Oberfläche wird durch die Krümmung des Unterteils 4 das Gewebe geglättet, woraufhin der Klebstoff abgegeben wird. Durch die konisch sich verbreiternde Form des Gehäuses und eines sich stetig verkleinernden Gesamtquerschnittes einerseits und durch das Anbringen von Leitstegen andererseits ergibt sich ein stetiger gleichmässiger Fluss des Klebstoffes auf die geglättete Unterlage, wobei die Höhe des Austragschlitzes Sowoehl von der Viskosität als auch vom erforderlichen Austragvolumen abhängig ist. Bei bestimmten Anwendungen kann die Länge des vorstehenden Teils 6A eine Rolle spielen.

Ausgehend vom oben dargestellten Ausführungsbeispiel gemäss den Figuren 1 - 3 sind für die Anwendung in der Medizinaltechnik viele Variationsmöglichkeiten gegeben.

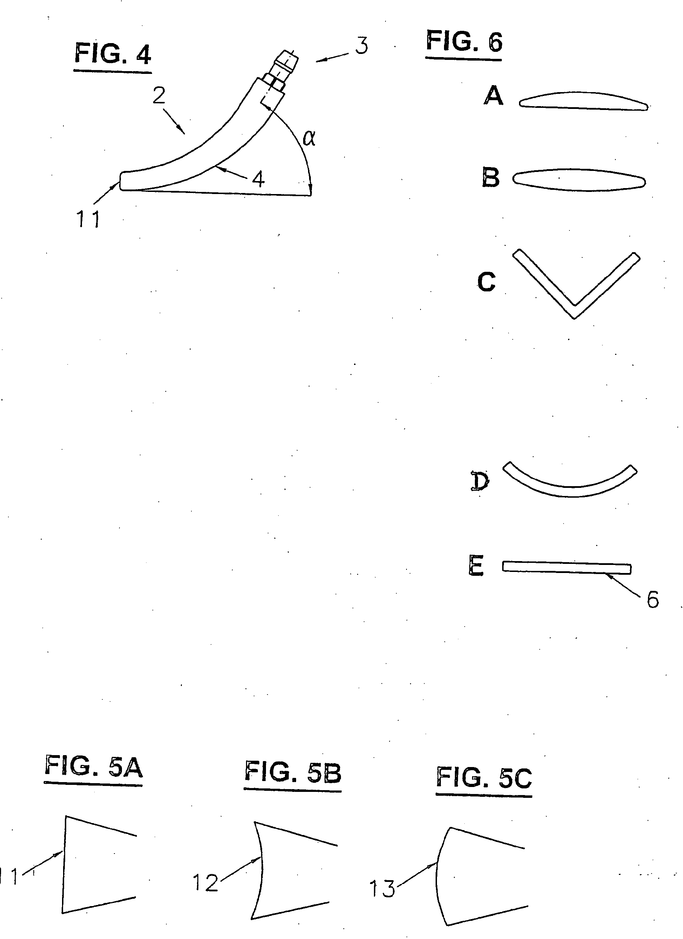

Gemäss Figur 4 ist das Gehäuse 2 stetig gekrümmt. Dabei kann der Winkel α zwischen der Längsachse des Anschlussteils 3 und dem Auslassende 11 der Unterseite zwischen 5° und 90° betragen, vorzugsweise zwischen 10° und 90°.

In den Figuren 5A bis 5C ist dargestellt, dass das den Austragschlitz aufweisende Auslassende 11 gerade wie gemäss Figur 1, das Auslassende 12 konkav wie in Figur 5B oder das Auslassende 13 konvex wie in Figur 5C sein kann.

Die Figuren 6A bis 6G zeigen verschiedene mögliche Querschnitte des Austragschlitzes, wobei Figur 6F dem Querschnitt von Austragschlitz 6 entspricht.

Je nach Anwendungszweck ist eine Variationsbreite des Austragschlitzquerschnittes, der Dimensionen der Leitstege etc. im unteren Millimeterbereich möglich.

## Patentansprüche

1. Auftragvorrichtung (1) für ein Austraggerät zum Verkleben eines Substrats, mit einem Anschlussteil (3) an einen Mischer oder an das Austraggerät selbst sowie einem Austragschlitz (6), wobei die dem zu verklebenden Substrat zugewandte Unterseite (4A) des Gehäuses (2) der Auftragvorrichtung (1) mit der Längsachse des Anschlussteils (3) einen Winkel (α) bildet, **dadurch gekennzeichnet, dass**:
- die Auftragvorrichtung (1) für die Medizinaltechnik bestimmt ist,
- das zu verklebende Substrat ein Gewebe ist,
- das Gehäuse (2) der Auftragvorrichtung (1) aus einem unteren Teil (4) und einem oberen Teil (5) besteht,
- die dem zu verklebenden Gewebe zugewandte Unterseite (4A) des Gehäuses (2) gekrümmt ist, wobei die Krümmung im wesentlichen stetig ist,
- der Austragschlitz (6) länglich ist und das dem Anschlussteil (3) entgegengesetzte Ende des Gehäuses (2) bildet, und
- sich das Gehäuse (2) zum länglichen Austragschlitz (6) hin konisch verbreitert.

2. Auftragvorrichtung (1) nach Anspruch 1, **dadurch gekennzeichnet, dass** der Winkel (α) zwischen 5° und 90° beträgt.

3. Auftragvorrichtung (1) nach Anspruch 1 oder 2, **dadurch gekennzeichnet, dass** das dem zu verklebenden Gewebe zugewandte, untere Teil (4) des Gehäuses (2) der Vorrichtung an seiner inneren Oberfläche Leitstege (7) aufweist.

4. Auftragvorrichtung (1) nach einem der Ansprüche 1 bis 3, **dadurch gekennzeichnet, dass** das vom zu verklebenden Gewebe abgewandte, obere Teil (5) des Gehäuses (2) der Vorrichtung länger ist als das dem zu verklebenden Gewebe zugewandte untere Teil (4) des Gehäuses (2) der Vorrichtung.

5. Auftragvorrichtung (1) nach einem der Ansprüche 1 bis 4, **dadurch gekennzeichnet, dass** die Krümmung kreisbogenförmig ist.

6. Auftragvorrichtung (1) nach einem der Ansprüche 1 bis 5, **dadurch gekennzeichnet, dass** das den Austragschlitz (6) aufweisende Auslassende gerade (11), konkav (12), oder konvex (13) geformt ist.

7. Auftragvorrichtung (1) nach einem der Ansprüche 1 bis 6, **dadurch gekennzeichnet, dass** der Querschnitt des Austragschlitzes (6) rechteckig, abgewinkelt, teilweise oder ganz bogenförmig oder oval sein kann.

## Claims

1. Applicator (1) for a dispensing appliance for gluing a substrate, including an adapter (3) for connection to a mixer or to the dispensing appliance itself as well as a dispensing slot (6), the underside (4A) of the enclosure (2) of the applicator (1) that is turned towards the substrate to be glued forming an angle (α) with the longitudinal axis of the adapter (3), **characterised in that**:
- the applicator (1) is intended for medicinal technique,
- the substrate that is to be glued is a tissue,
- the enclosure (2) of the applicator (1) is composed of a lower portion (4) and an upper portion (5),
- the side (4A) of the enclosure (2) that is turned towards the glued tissue is curved, the curvature being essentially continuous,
- the dispensing slot (6) is oblong and forms the end of the enclosure (2) opposite the adapter (3),
- the enclosure (2) is conically enlarged towards the oblong dispensing slot (6).

2. Applicator (1) according to claim 1, **characterised in that** the angle (α) is comprised between 5° and 90°.

3. Applicator (1) according to claim 1 or 2, **characterised in that** the lower portion (4) of the enclosure (2) of the device, which is turned towards the glued tissue, is provided with guiding walls (7) on its inner surface.

4. Applicator (1) according to any one of claims 1 to 3, **characterised in that** the upper portion (5) of the enclosure (2) of the device, which is turned away from the glued tissue, is longer than the lower portion (4) of the enclosure (2) of the device that is turned towards the glued tissue.

5. Applicator (1) according to any one of claims 1 to 4, **characterised in that** the curvature is circle arc shaped.

6. Applicator (1) according to any one of claims 1 to 5, **characterised in that** the dispensing end provided with the dispensing slot (6) is straight (11), concave (12), or convex (13) in shape.

7. Applicator (1) according to any one of claims 1 to 6, **characterised in that** the cross-section of the dispensing slot (6) is rectangular, angled, partly or entirely arc-shaped, or oval.

## Revendications

1. Dispositif à étendre (1) pour un applicateur destiné au collage d'un substrat, comprenant une partie de raccordement (3) à un mélangeur ou à l'applicateur lui-même, ainsi qu'une fente de distribution (6), le côté inférieur (4A) du boîtier (2) du dispositif à étendre (1), en regard du substrat à coller, formant un angle (α) avec l'axe longitudinal de la partie de raccordement (3), **caractérisé en ce que**:
- le dispositif à étendre (1) est destiné à la technique médicale,
- le substrat à coller est un tissu,
- le boîtier (2) du dispositif à étendre (1) est composé d'une partie inférieure (4) et d'une partie supérieure (5),
- le côté (4A) du boîtier (2) en regard du tissu à coller est courbé, la courbure étant essentiellement uniforme,
- la fente de distribution (6) est allongée et forme l'extrémité du boîtier (2) opposée à la partie de raccordement (3), et que
- le boîtier (2) s'élargit de façon conique vers la fente de distribution (6) allongée.

2. Dispositif à étendre (1) selon la revendication 1, **caractérisé en ce que** l'angle (α) est compris entre 5° et 90°.

3. Dispositif à étendre (1) selon la revendication 1 ou 2, **caractérisé en ce que** la partie inférieure (4) du boîtier (2) du dispositif, en regard du tissu à coller, présente des nervures de guidage (7) sur sa surface intérieure.

4. Dispositif à étendre (1) selon l'une quelconque des revendications 1 à 3, **caractérisé en ce que** la partie supérieure (5) du boîtier (2) du dispositif, opposée au tissu à coller, est plus longue que la partie inférieure (4) du boîtier (2) du dispositif en regard du tissu à coller.

5. Dispositif à étendre (1) selon l'une quelconque des revendications 1 à 4, **caractérisé en ce que** la courbure est en arc de cercle.

6. Dispositif à étendre (1) selon l'une quelconque des revendications 1 à 5, **caractérisé en ce que** l'extrémité de décharge présentant la fente de distribution (6) est de forme droite (11), concave (12) ou convexe (13).

7. Dispositif à étendre (1) selon l'une quelconque des revendications 1 à 6, **caractérisé en ce que** la coupe transversale de la fente de distribution (6) peut être rectangulaire, coudée, partiellement ou entièrement arquée ou ovale.
